# EUROPEAN PATENT APPLICATION

(11) **EP 2 248 860 A1**
(43) Date of publication of application: **10.11.2010**
(21) Application number: 08800707.5
(22) Date of filing: 29.08.2008
(51) Int. Cl.: C09B 62/01, C07C 245/22

(54) **AN ORANGE ACTIVE DYE AND THE PREPARATION THEREOF**

(30) Priority: 22.01.2008 CN 200810052129
(71) Applicant: Tianjin Dek Chemical Co., Ltd, Tianjin 300163 (CN)
(72) Inventor: ZHANG, Xinghua, Tianjin 300163 (CN); LIU, Wenxiang, Tianjin 300163 (CN)
(74) Representative: Bohnenberger, Johannes
(86) International application number: PCT/CN2008/072195
(87) International publication number: WO 2009/092211

(57) **Abstract**

The present invention provides an orange active dye and the preparation method thereof. The coupling constituent is sodium 2,4-diamino-benzene sulfonate, and the diazo constituent is 2-naphthylamine-disulfonic acid and β-hydroxyethylsulfurylsulfate aniline. The product is obtained by coupling twice, filtering, drying and packing. The active orange dye has an excellent solubility, and has a rate of exhaustion of 96% in dyeing. It is apparently superior to acid dyes, and greatly reduces the difficulty of environmental treatment.

## Description

### Technique field

The prevention provides an active dye and the preparation method thereof, especially an orange active dye and the preparation method thereof.

### Technique background

Active dyes have various remarkable properties such as vivid color and lustre, excellent application property, simple processing, and so on, however, there are some limitation in the active dyes applicable to protein fiber, particularly in the economic active dyes capable of replacing acid dye. An active dye applicable to protein fiber is produced, which has good compatibleness with black active dye resulting in an economic black active dye with vivid color and lustre. Due to the widespread market potential, its structure is important. For acid dye, the color of the residual liquid after dyeing is dark, wet cohesional strength is poor, and the environmental treatment is difficult. Furthermore, strong-acid type dye is harmful to fiber during dyeing in the strong-acid medium, and the feeling of the resulted fabric is dissatisfactory. Along with the improvement of environmental awareness, active dye is realized to be more valuable than acid dye in cost and environmental treatment.

### Detailed description of the disclosure

An object of the present invention is to provide an orange active dye with high dyeing rate, excellent solubility, outstanding application performance, advantageous usableness, reduced pollution, and ability of replacing acid dye and mordant dye, as well as the preparation method thereof.

For solving the above-mentioned problem, the embodiment of the present invention is: An orange active dye, having the following general formula 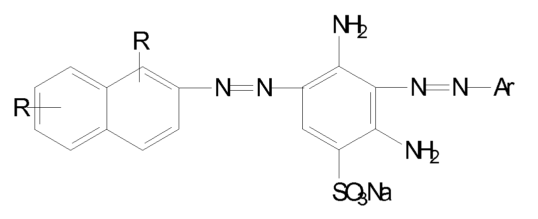 where R is —SO₃H or —SO₃Na, the number of R is one or two,

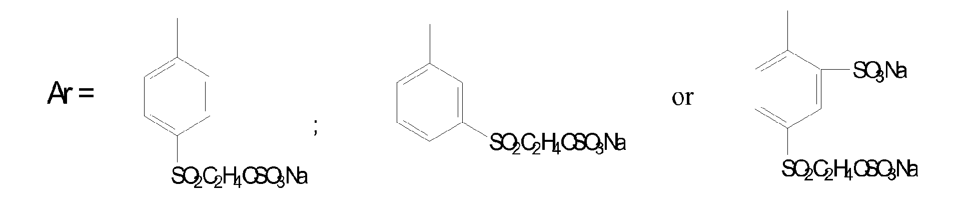

A preparation method of the orange active dye, comprising the following steps:
a. Diazo reaction: 2-naphthylamine-disulfonic acid is added into water and pulped under agitation at room temperature. Ice is added. Then, both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-disulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: β-hydroxyethylsulfurylsulfate aniline is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of β-hydroxyethylsulfurylsulfate aniline prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

A preparation method of the orange active dye, comprising the following steps:
a. Diazo reaction: 2-naphthylamine-sulfonic acid is added into water and pulped under agitation at room temperature. Ice is added. Then both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-sulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: β-hydroxyethylsulfurylsulfate aniline is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of β-hydroxyethylsulfurylsulfate aniline prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

A preparation method of the orange active dye, comprising the following steps:
a. Diazo reaction: 2-naphthylamine-disulfonic acid is added into water and pulped under agitation at room temperature. Ice is added. Then, both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-disulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: Sodium 2-amino-5-β-hydroxyethylsulfurylsulfate benzene sulfonate is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of sodium 2-amino-5-β-hydroxyethylsulfurylsulfate benzene sulfonate prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

A preparation method of the orange active dye, comprising the following steps:
a. Diazo reaction: 2-naphthylamine-sulfonic acid is added into water and pulped under agitation at room temperature. Ice is added. Then, both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-sulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: Sodium 2-amino-5-β-hydroxyethylsulfurylsulfate benzene sulfonate is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of sodium 2-amino-5-β-hydroxyethylsulfurylsulfate benzene sulfonate prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

The adjustment of the pH value by using inorganic alkali in step (d) is carried out through the successive steps consisting of the adjustment of pH to 4 with sodium carbonate and to 5-6.5 with sodium bicarbonate.

The advantages of the present invention include that the active orange dye has an excellent solubility, and has a rate of exhaustion of 98% in dyeing. It is apparently superior to acid dyes, and greatly reduces the difficulty of environmental treatment, resulting in better social benefit.

### Brief description of the drawings

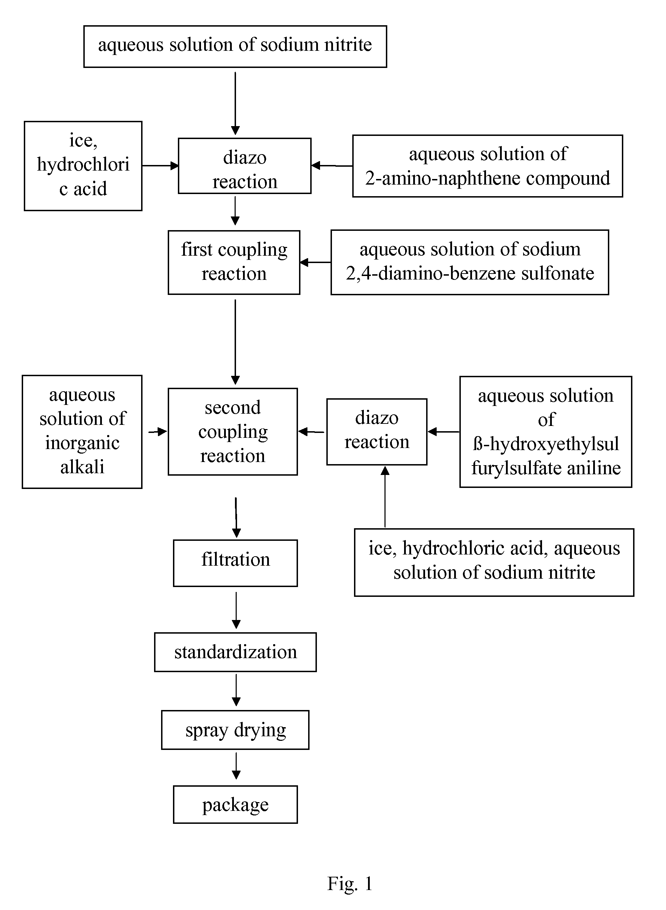

Fig. 1 is a process flow diagram of a preparation method of the orange active dye provided in the present invention.

### Embodiment

Accompanying with drawing and examples, the invention is further described in details, as following:
An orange active dye of the present invention, having the following general formula 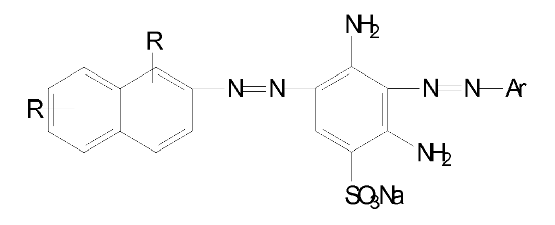 Where R is —SO₃H or —SO₃Na, the number of R is one or two,
   or

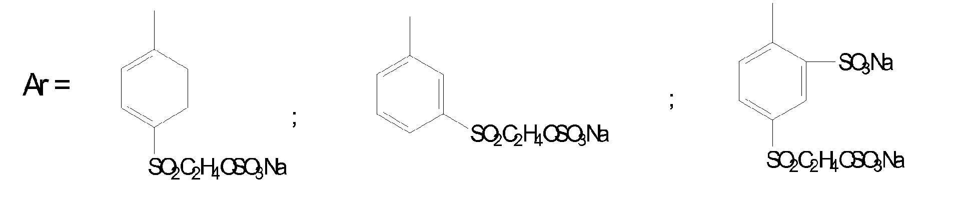

As illustrated in Fig.1, a preparation method of the orange active dye provided in the invention comprises the following steps:
a. Diazo reaction: 2-naphthylamine-disulfonic acid is added into water and pulped under agitation at room temperature. Ice is added. Then, both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-disulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: β-hydroxyethylsulfurylsulfate aniline is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of β-hydroxyethylsulfurylsulfate aniline prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

In the present invention, 2-naphthylamine-disulfonic acid can be substituted by 2-naphthylamine-sulfonic acid, and β-hydroxyethylsulfurylsulfate aniline can be substituted by sodium 2-amino-5-β-hydroxyethylsulfurylsulfate benzene sulfonate.

The adjustment of the pH value by using inorganic alkali in step (d) is carried out through the successive steps consisting of the adjustment of pH to 4 with sodium carbonate and to 5-6.5 with sodium bicarbonate.

As follows, more detailed description accompanying with examples is provided.

### Example 1

An active orange dye of the following formula: 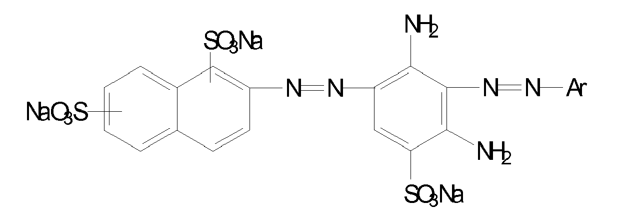 where

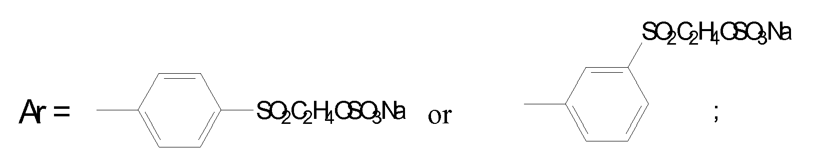

### Preparation method:

An example where 2-naphthylamine-1,5-disulfonic acid and p-β-hydroxyethylsulfurylsulfate aniline were used as the diazo constituents was explained. If meta-β-hydroxyethylsulfurylsulfate aniline was used as another diazo constituent, the related dissolution way of the material and the amount of hydrochloric acid could be different, while the other procedures were similar.

### 1. Proportion of raw materials:

| Raw materials | Molar mass | Molar ratio | 100% dosage | Water(t) |
|---|---|---|---|---|
| sodium 2,4-diamino-benzene sulfonate | 210.2 | 1.00 | 210.2 | 0.7 |
| 2-naphthylamine-1,5-disulfonic acid | 303.3 | 1.01 | 306.3 | 0.7 |
| β-hydroxyethylsulfurylsulfate aniline | 281.3 | 1.01 | 284.1 | 0.7 |
| sodium nitrite | 69 | 2.03 | 140.2 | 0.468 |
| hydrochloric acid | 36.5 | 2.93 | 106.9 | -- |
| sodium carbonate | 106 | Based on pH value | | |
| sodium bicarbonate | 84 | Based on pH value | | |
| ice | 1200 | | | |

### 2. Preparation steps:

(1) 2-naphthylamine-1,5-disulfonic acid was added into 700L water and pulped under agitation at room temperature.
(2) 70.1 Kg of sodium nitrite(100%) were dissolved in 234L water so as to generate a solution with the concentration of about 30%.
(3)About 400 Kg of ice and 159.7 Kg of technical hydrochloric acid(30%) were introduced into the aqueous solution of 2-naphthylamine-1,5-disulfonic acid. The temperature of the liquor was adjusted with ice to 0-6°C, then aqueous solution of sodium nitrite was added in. The pH value of the obtained liquor is maintained to be 1-3, and the amount of nitrous acid is slightly excess. The reaction was allowed to proceed under agitation for two hour. Subsequently, the residual nitrous acid was removed during the following heat preservation.
(4) The dissolution of sodium 2,4-diamino-benzene sulfonate: Sodium 2,4-diamino-benzene sulfonate was added into 700L water and pulped to dissolve at room temperature.
(5) First coupling reaction: The diazo salt of 2-naphthylamine-1,5-disulfonic acid was introduced into the aqueous solution of sodium 2,4-diamino-benzene sulfonate. Under agitation, with temperature of 5-15°C and pH value of 1-3, the reaction lasted one hour. The end of the first coupling reaction is measured by using spectrophotometer.
(6) The dissolution of β-hydroxyethylsulfurylsulfate aniline: β-hydroxyethylsulfurylsulfate aniline was added into 700L water and pulped under agitation at room temperature.
(7) 70.1 Kg of sodium nitrite(100%) were dissolved in 234L water so as to generate a solution with the concentration of about 30%.
(8) Diazo reaction: 400 Kg of ice and 196.6 Kg of technical hydrochloric acid(30%) were introduced into the aqueous solution of p-β-hydroxyethylsulfurylsulfate aniline. The temperature of the liquor was adjusted with ice to 0-6°C, then aqueous solution of sodium nitrite was added in. The pH value of the obtained liquor is maintained to be 1-3, and the amount of nitrous acid is slightly excess. The reaction was allowed to proceed under agitation for two hour. Subsequently, the residual nitrous acid was removed during the following heat preservation.
(9) Second coupling reaction: The diazo salt of p-β-hydroxyethylsulfurylsulfate aniline was added into first coupling reaction liquor. Under agitation the pH value was adjusted with sodium carbonate solution to 4 and then with sodium bicarbonate solution to 5-6.5. At 5-15°C, the coupling reaction lasted one hour.
(10) Filtration: After the reaction, the as-produced liquor was filtered. The filtrate was collected into the tank, and the filter residue was treated through centralized processing.
(11) Standardization: The collected filtrate was standardized.
(12) Spray drying: The standardized liquor was spray dried.
(13) Package: The as-produced dry material was packaged as products.

### Example 2

An active orange dye of the following formula:

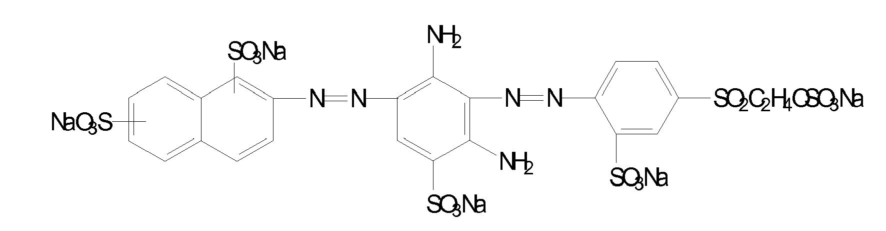

### Preparation method:

An example where 2-naphthylamine-4,8-disulfonic acid and sodium 2-amino-5-β-hydroxyethylsulfurylsulfate benzene sulfonate were used as the diazo constituents was explained.

### 1. Proportion of raw materials:

| Raw materials | Molar mass | Molar ratio | 100% dosage | Water(t) |
|---|---|---|---|---|
| sodium 2,4-diamino-benzene sulfonate | 210.2 | 1.00 | 210.2 | 0.7 |
| 2-naphthylamine-4,8-disulfonic acid | 303.3 | 1.01 | 306.3 | 0.8 |
| sodium 2-amino-5-β-hydroxyethylsulfurylsulfate benzene sulfonate | 361.4 | 1.01 | 365 | 0.7 |
| sodium nitrite | 69 | 2.03 | 140.2 | 0.468 |
| hydrochloric acid | 36.5 | 3.53 | 129 | -- |
| sodium carbonate | 106 | Based on pH value | | |
| sodium bicarbonate | 84 | Based on pH value | | |
| ice | 1200 | | | |

### 2. Preparation steps:

(1) 2-naphthylamine-4,8-disulfonic acid was added into 700L water and pulped under agitation at room temperature.
(2) 70.1 Kg of sodium nitrite(100%) were dissolved in 234L water so as to generate a solution with the concentration of about 30%.
(3) About 400 Kg of ice and 159.7 Kg of technical hydrochloric acid(30%) were introduced into the aqueous solution of sodium 2-naphthylamine-4,8-disulfonic acid. The temperature of the liquor was adjusted with ice to 0-6°C, then aqueous solution of sodium nitrite was added in. The pH value of the obtained liquor is maintained to be 1-3, and the amount of nitrous acid is slightly excess. The reaction was allowed to proceed under agitation for two hour. Subsequently, the residual nitrous acid was removed during the following heat preservation.
(4) The dissolution of sodium 2,4-diamino-benzene sulfonate: Sodium 2,4-diamino-benzene sulfonate was added into 800L water and pulped to dissolve at room temperature.
(5) First coupling reaction: The diazo salt of 2-naphthylamine-4,8-disulfonic acid was introduced into the aqueous solution of sodium 2,4-diamino-benzene sulfonate. Under agitation, with temperature of 5-15°C and pH value of 1-4, the reaction lasted one hour. The end of the first coupling reaction is measured by using spectrophotometer.
(6) The dissolution of sodium 2-amino-5-β-hydroxyethylsulfurylsulfate benzene sulfonate: sodium 2-amino-5-β-hydroxyethylsulfurylsulfate benzene sulfonate was added into 700L water and pulped under agitation at room temperature.
(7) 70.1 Kg of sodium nitrite(100%) were dissolved in 234L water so as to generate a solution with the concentration of about 30%.
(8) Diazo reaction: 400 Kg of ice and 270.3 Kg of technical hydrochloric acid(30%) were introduced into the aqueous solution of sodium 2-amino-5-β-hydroxyethylsulfurylsulfate benzene sulfonate. The temperature of the liquor was adjusted with ice to 0-6°C, then aqueous solution of sodium nitrite was added in. The pH value of the obtained liquor is maintained to be 1-3, and the amount of nitrous acid is slightly excess. The reaction was allowed to proceed under agitation for two hour. Subsequently, the residual nitrous acid was removed during the following heat preservation.
(9) Second coupling reaction: The diazo salt of sodium 2-amino-5-β-hydroxyethylsulfurylsulfate benzene sulfonate was added into first coupling reaction liquor. Under agitation the pH value was adjusted with sodium carbonate solution to 4 and then with sodium bicarbonate solution to 5-6.5. At 5-15°C, the coupling reaction lasted one hour.
(10) Filtration: After the reaction, the as-produced liquor was filtered. The filtrate was collected into the tank, and the filter residue was treated through centralized processing.
(11) Standardization: The collected filtrate was standardized.
(12) Spray drying: The standardized liquor was spray dried.
(13) Package: The as-produced dry material was packaged as products.

The present invention is not limited to the above-mentioned examples. It will be understood that variations and modifications can be effected within the scope of the invention as described above, by a person of ordinary skill in the art without departing from the scope of the invention.

## Claims

1. An orange active dye, **characterized in that** it has the following general formula 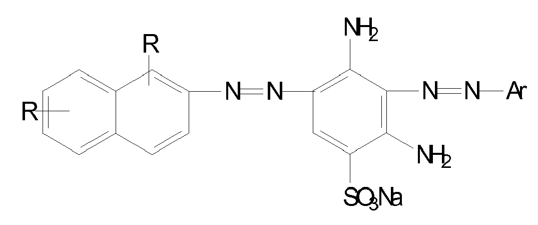 where R is —SO₃H or —SO₃Na, the number of R is one or two,

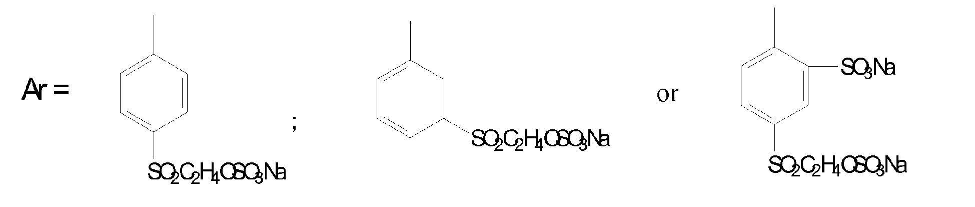

2. A preparation method of the orange active dye of claim 1, comprising the following steps:
a. Diazo reaction: 2-naphthylamine-disulfonic acid is added into water and pulped under agitation at room temperature. Ice is added. Then, both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-disulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: β-hydroxyethylsulfurylsulfate aniline is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of β-hydroxyethylsulfurylsulfate aniline prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

3. A preparation method of the orange active dye of claim 1, comprising the following steps:
a. Diazo reaction: 2-naphthylamine-sulfonic acid is added into water and pulped under agitation at room temperature. Ice is added. Then both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-sulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: β-hydroxyethylsulfurylsulfate aniline is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of β-hydroxyethylsulfurylsulfate aniline prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

4. A preparation method of the orange active dye of claim 1, comprising the following steps:
a. Diazo reaction: 2-naphthylamine-disulfonic acid is added into water and pulped under agitation at room temperature. Ice is added. Then, both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-disulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: Sodium 2-amino-5-β- hydroxyethylsulfurylsulfate benzene sulfonate is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of sodium 2-amino-5-β-hydroxyethylsulfurylsulfate benzene sulfonate prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

5. A preparation method of the orange active dye of claim 1, comprising the following steps:
a. Diazo reaction: 2-naphthylamine-sulfonic acid is added into water and pulped under agitation at room temperature. Ice is added. Then, both hydrochloric acid and aqueous solution of sodium nitrite are introduced in the solution at 0-10°C. The pH value of the obtained reaction liquor is maintained to be 1-3, the amount of nitrous acid is slightly excess. After two hours of reaction under agitation, the reaction is finished and the residual nitrous acid is removed.
b. First coupling reaction: Sodium 2,4-diamino-benzene sulfonate is dissolved in water at room temperature. Then the diazo reaction liquor of 2-naphthylamine-sulfonic acid obtained in step (a) is introduced in. With pH value of 1-4 and temperature of 5-17°C, the coupling reaction lasts one hour under agitation. The end of the coupling reaction is measured by using spectrophotometer.
c. Diazo reaction: Sodium 2-amino-5-β-hydroxyethylsulfurylsulfate benzene sulfonate is added into water and pulped under agitation at room temperature. Then ice, hydrochloric acid, and aqueous solution of sodium nitrite are introduced in the solution. The temperature is 0-10°C, the pH value of the obtained reaction liquor is maintained to be 1-3, and the amount of nitrous acid is slightly excess. After two hours of reaction under agitation, the reaction is accomplished and the residual nitrous acid is removed.
d. Second coupling reaction: The diazo reaction liquor of sodium 2-amino-5-β-hydroxyethylsulfurylsulfate benzene sulfonate prepared in step (c) is added into first coupling reaction liquor obtained in step (b). Under agitation the pH value is adjusted to be 5-6.5 by using inorganic alkali. At 5-17°C, the coupling reaction lasts one hour.
e. Commercialization: After the reaction, the as-produced liquor is filtered. The filtrate is collected into the liquid tank, and the filter residue is treated through centralized processing. Then the filtrate is standardized, spray-dried, and packaged.

6. Preparation method of any of claim 2-5, **characterized in that** the adjustment of the pH value by using inorganic alkali in said step (d) is carried out through the successive steps consisting of the adjustment of pH to 4 with sodium carbonate and to 5-6.5 with sodium bicarbonate.
